# EUROPEAN PATENT APPLICATION

(11) **EP 4 144 321 A1**
(43) Date of publication of application: **08.03.2023**
(21) Application number: 21797388.2
(22) Date of filing: 27.04.2021
(51) Int. Cl.: A61B 34/30, F16C 29/04

(54) **GUIDE RAIL DEVICE AND MEDICAL ROBOT**

(30) Priority: 29.04.2020 CN 202010354301
(71) Applicant: Suzhou Kangduo Robot Co., Ltd., Suzhou, Jiangsu 215153 (CN)
(72) Inventor: WANG, Jianguo, Suzhou, Jiangsu 215153 (CN); WANG, Xiaowei, Suzhou, Jiangsu 215153 (CN)
(74) Representative: Zaboliene, Reda
(86) International application number: PCT/CN2021/090205
(87) International publication number: WO 2021/218955

(57) **Abstract**

The invention provides a guide rail device and a medical robot, and relates to the technical field of medical instruments. The guide rail device comprises a curvilinear guide rail and a slider, wherein the slider is slidably connected to the curvilinear guide rail; the bottom of the slider is adapted to connecting with the medical robot; the curvilinear guide rail comprises a first arc section guide rail and a second arc section guide rail; and an arc opening direction of the first arc section guide rail is opposite to that of the second arc section guide rail. The curvilinear guide rail is arranged at an altitude above a patient to be operated, so as to solve problems of the existing laparoscopic surgical robot with a floor-standing structure, such as inflexible walking, inconvenient operation, easy blocking of the view and occupation for a lot of ground space.

## Description

### Technical Field

The invention relates to the technical field of medical instruments, in particular to a guide rail device and a medical robot.

### Background Art

Compared with traditional surgery, laparoscopic surgery is highly popular with patients; especially surgical scars are small and meet aesthetic requirements, so that young patients are more willing to accept the laparoscopic surgery; and the laparoscopic surgery as a minimally invasive surgery is a general trend and pursuit goal of surgical development.

Laparoscopic surgical robots are important equipment for the laparoscopic surgery; the existing medical robots represented by the laparoscopic surgical robots generally have a floor-standing structure, which has disadvantages of inflexible walking, inconvenient operation, easy blocking of the view, occupation for a lot of ground space and the like.

### Summary of the Invention

The invention aims to solve problems that the existing medical robots represented by laparoscopic surgical robots generally have a floor-standing structure, and the structure has disadvantages of inflexible walking, inconvenient operation, easy blocking of the view, occupation for a lot of ground space and the like.

To solve the problems, the invention provides a guide rail device, comprising:
a curvilinear guide rail; and
a slider slidably connected to the curvilinear guide rail, wherein the bottom of the slider is adapted to connecting with a medical robot;
the curvilinear guide rail comprises a first arc section guide rail and a second arc section guide rail; and an arc opening direction of the first arc section guide rail is opposite to that of the second arc section guide rail.

Further, the curvilinear guide rail comprises a first linear guide rail, a second linear guide rail and a third linear guide rail which are parallel to one another; the first linear guide rail, the first arc section guide rail, the second linear guide rail, the second arc section guide rail and the third linear guide rail are sequentially connected; an opening direction of the first arc section guide rail faces one end of the second linear guide rail; and an opening direction of the second arc section guide rail faces the other end of the second linear guide rail.

Further, the curvilinear guide rail comprises a guide rail body and a supporting rail plate; the supporting rail plate is connected with the guide rail body; the supporting rail plate has an elongated plate-like structure and extends along a length direction of the curvilinear guide rail; two supporting rail plates are provided and respectively arranged at two sides of the guide rail body;

The slider comprises a sliding mechanism, which is composed of two bearing wheels; the two bearing wheels are respectively arranged at the tops of the two supporting rail plates; and the guide rail body is arranged between the two bearing wheels, so as to prevent the slider from being separated from the curvilinear guide rail.

Further, the slider comprises:
a fixed base plate; and
two rotating blocks, which are rotationally connected to the top of the fixed base plate respectively; the bearing wheels are arranged at the rotating blocks; and the supporting rail plates are arranged between the bearing wheels and the rotating blocks.

Further, the bearing wheels are rotationally connected with the rotating blocks.

Further, the slider comprises a stable moving mechanism; the stable moving mechanism is composed of two retaining wheels; the retaining wheels are arranged at the rotating blocks; rims of the retaining wheels are provided with annular wheel grooves; and two side edges of the supporting rail plates are respectively arranged in the wheel grooves of the two retaining wheels, to prevent the slider from shaking while moving.

Further, the retaining wheels are rotationally connected with the rotating blocks.

Further, the curvilinear guide rail comprises a rack; the rack extends along the length direction of the supporting rail plates; the slider further comprises a gear and a driving motor; the driving motor is used for driving the gear to rotate; and the gear is meshed with the rack.

Further, a brake is arranged at the top of the slider; and the brake is adopted to stopping the slider from moving along the curvilinear guide rail.

In addition, the invention further provides a medical robot, comprising the guide rail device.

Further, the medical robot comprises a robot body; and the robot body is connected with the slider of the guide rail device.

Further, the robot body comprises a lifting rod, rotating arms, telescopic rods and surgical manipulators, which are sequentially arranged from top to bottom; and the top of the lifting rod is connected with the bottom of the slider.

Further, the curvilinear guide rail and the slider of the guide rail device and the robot body are sequentially arranged from top to bottom.

The medical robot plays the same role in the utility model as the guide rail device, so the medical robot will not be explained.

Compared with the prior art, the guide rail device provided by the invention has but is not limited to the following technical effects:
the curvilinear guide rail is arranged at an altitude above a patient to be operated, so the curvilinear guide rail in such a state will not block the view of patients and doctors, nor occupy the space on the ground; by adopting such a "curvilinear" design that the slider slides along the curvilinear guide rail, the slider is capable of sliding in a two-dimensional plane together with the robot body by just moving the slider to an appropriate position on the two-dimensional plane, thereby facilitating operation; and in such a suspending arrangement case, the slider is connected with the medical robot, so that the medical robot will not be hindered by obstacles (such as hospital beds and operating tables) and is able to walk flexibly, thereby solving problems that the existing medical robots represented by laparoscopic surgical robots generally have a floor-standing structure, and the structure has disadvantages of inflexible walking, inconvenient operation, easy blocking of the view, occupation for a lot of ground space and the like.

### Brief Description of the Drawings

Figure 1 is a schematic structural diagram of a guide rail device according to embodiments of the invention;
Figure 2 is a schematic structural diagram of a slider according to embodiments of the invention;
Figure 3 is an enlarged view of B in Figure 1;
Figure 4 is an enlarged view of A in Figure 1; and
Figure 5 is a schematic structural diagram of a meshing state of a gear and a rack according to embodiments of the invention.

Description of reference numerals:
1-curvilinear guide rail, 101-first linear guide rail, 102-first arc section guide rail, 103-second linear guide rail, 104-second arc section guide rail, 105-third linear guide rail, 11-supporting rail plate, 111-side edge, 12-rack, 13-guide rail body;
2-slider, 21-fixed base plate, 22-rotating block, 23-retaining wheel, 231-wheel groove, 24-bearing wheel, 25-brake, 26-gear, 27-driving motor; and
3-robot body, 31-lifting rod, 32-arm rotating bracket, 33-rotating arm, 34-telescopic rod, 35-surgical manipulator.

### Detailed Description of the Invention

To make objects, features and advantages of the invention plainer and more understandable, embodiments of the invention will be described in detail below with reference to the accompanying drawings.

In description of the invention, it should be understood that azimuths or positional relationships indicated by terms "upper", "lower", "front", "rear" and the like are based on azimuths or positional relationships shown in the accompanying drawings, are only used for facilitating the description of the invention and simplifying the description, instead of indicating or implying that the referred devices or elements must have specific directions and be constructed and operated in specific directions, and therefore cannot be understood as a limitation to the invention.

Moreover, a Z-axis in the accompanying drawings represents a vertical direction, i.e., an upper-and-lower position, and a positive direction of the Z-axis (i.e., an arrow direction of the Z-axis) represents the upper, while a negative direction of the Z-axis (i.e., a direction opposite to the positive direction of the Z-axis) represents the lower;

An X-axis in the accompanying drawings represents a longitudinal direction of a horizontal plane and is perpendicular to the Z-axis, and the positive direction of the X-axis (i.e., an arrow direction of the X-axis) represents a front side, while the negative direction of the X-axis (i.e., a direction opposite to the positive direction of the X-axis) represents a rear side;

A Y-axis in the accompanying drawings represents a transverse direction of the horizontal plane and is perpendicular to the Z-axis and the X-axis, and the positive direction of the Y-axis (i.e., an arrow direction of the Y-axis) represents a left side, and the negative direction of the Y-axis (i.e., a direction opposite to the positive direction of the Y-axis) represents a right side;

A plane formed by the X-axis and the Z-axis is a vertical plane.

Meanwhile, it should be noted that meanings of the Z-axis, Y-axis and X-axis are only used for facilitating the description of the invention and simplifying the description, instead of indicating or implying that the referred devices or elements must have specific directions and be constructed and operated in specific directions, and therefore cannot be understood as the limitation to the invention.

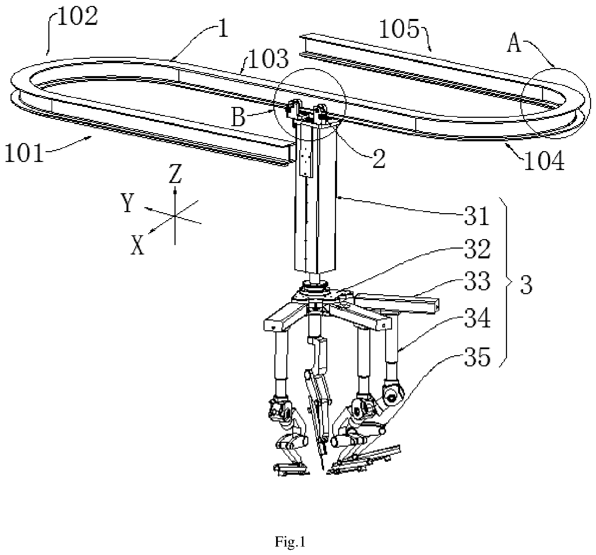

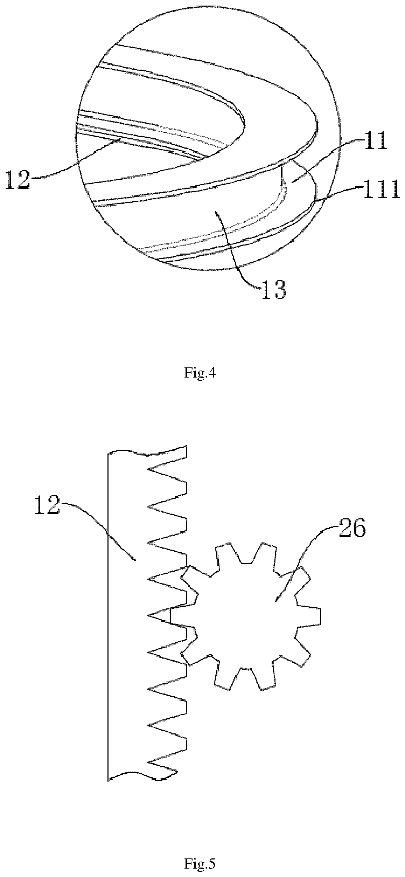

Referring to Figures 1-5, the present embodiment provides a guide rail device, comprising:
a curvilinear guide rail 1; and
a slider 2, which is matched with the curvilinear guide rail 1, i.e., slidably connected to the curvilinear guide rail 1, wherein the slider 2 is adapted to moving along the curvilinear guide rail 1; and the bottom of the slider 2 is adapted to connecting with a medical robot, such as a laparoscopic surgical robot or other medical robots.

Here, the curvilinear guide rail 1 may be arranged at an altitude, which refers to a position above an operating table or hospital bed; and more specifically, the curvilinear guide rail 1 is arranged above an operating region of a patient, and may be arranged on a roof or the top of the operating table.

The curvilinear guide rail 1 in such a state will not block the view of patients and doctors, nor occupy the space on the ground; by adopting such a "curvilinear" design that the slider 2 slides along the curvilinear guide rail 1, the slider 2 is capable of sliding in a two-dimensional plane together with the medical robot by just moving the slider 2 to an appropriate position on the two-dimensional plane, thereby facilitating operation; in such a suspending arrangement case, the medical robot connected with the bottom of the slider 2 will not be hindered by obstacles such as hospital beds and is able to walk flexibly, thereby solving problems that the existing laparoscopic surgical robots with a floor-standing structure have disadvantages of inflexible walking, inconvenient operation, easy blocking of the view, occupation for a lot of ground space and the like; the two-dimensional plane refers to an XY plane in Figure 1; certainly, the curvilinear guide rail 1 may be not only in a two-dimensional plane, but also in a three-dimensional space, i.e., the curvilinear guide rail 1 may also be a three-dimensional curvilinear guide rail; and in this case, the slider 2 is capable of moving in the three-dimensional space, not limited to the two-dimensional plane, and high in flexibility.

Meanwhile, it should be noted that the "arranged at" and "arranged in" include various connection modes such as fixed connection and detachable connection, and so do the "arranged at" and "arranged in" mentioned later in the present embodiment.

Referring to Figures 1-4, preferably, the curvilinear guide rail 1 comprises a first arc section guide rail 102 and a second arc section guide rail 104; and an arc opening direction of the first arc section guide rail 102 is opposite to that of the second arc section guide rail 104, i.e., the first arc section guide rail 102 and the second arc section guide rail 104 protrude toward different directions.

The first arc section guide rail 102 and the second arc section guide rail 104 are adopted to ensure that the curvilinear guide rail 1 is provided with at least two bends and the slider 2 is capable of changing the direction at least twice in a two-dimensional plane. Compared with the traditional linear guide rail, the curvilinear guide rail is more flexible and changeable in moving mode.

Referring to Figures 1-4, preferably, the curvilinear guide rail 1 further comprises a first linear guide rail 101, a second linear guide rail 103 and a third linear guide rail 105; the first linear guide rail 101, the first arc section guide rail 102, the second linear guide rail 103, the second arc section guide rail 104 and the third linear guide rail 105 are sequentially connected; the first arc section guide rail 102 protrudes toward one end of the second linear guide rail 103; and the second arc section guide rail 105 protrudes toward the other end of the second linear guide rail 103.

The first linear guide rail 101, the first arc section guide rail 102, the second linear guide rail 103, the second arc section guide rail 104 and the third linear guide rail 105 are sequentially connected, so that the curvilinear guide rail 1 may be an S-shaped guide rail with an attractive shape and is more practical when moving in a two-dimensional plane.

Referring to Figures 1-3, preferably, the curvilinear guide rail 1 comprises a guide rail body 13 and a supporting rail plate 11; the supporting rail plate 11 is connected with the guide rail body 13; for example, the supporting rail plate 11 may be connected to the bottom of the curvilinear guide rail 1; the supporting rail plate 11 has an elongated plate-like structure and extends along a length direction of the curvilinear guide rail 1; the slider 2 comprises a sliding mechanism, which is suspended on the supporting rail plate 11; and the sliding mechanism cooperates with the supporting rail plate 11 to prevent the slider 2 from being separated from the curvilinear guide rail 1.

The supporting rail plate 11 is arranged to provide a region for moving and supporting the slider 2; and the sliding mechanism ensures the slider 2 to move along the curvilinear guide rail 1 in a mode of suspending the slider on the supporting rail plate 11, thereby preventing the slider 2 from being separated from the curvilinear guide rail 1.

Referring to Figures 1-4, preferably, a supporting rail plate 11 may also be arranged at the top of the guide rail body 13; the curvilinear guide rail 1 may be fixed on the roof by the supporting rail plate 11 arranged at the top; and the supporting rail plate 11 at the top provides an effective region for fixing the curvilinear guide rail 1.

Referring to Figures 1-4, preferably, the slider 2 further comprises:
a fixed base plate 21; and
two rotating blocks 22, which are rotationally connected to the top of the fixed base plate 21 respectively; the bearing wheels 24 are arranged at the rotating blocks 22; and the supporting rail plates 11 are arranged between the bearing wheels 24 and the rotating blocks 22.

The two bearing wheels 24 are respectively arranged on the supporting rail plates 11 on two sides of the curvilinear guide rail 1 body; the curvilinear guide rail body 13 guides and limits the two bearing wheels 24 to ensure that the bearing wheels will not be separated from the curvilinear guide rail 1; and it should be understood that the bearing wheels 24 not only play a guiding role on the slider 2, but also play a bearing role on a corresponding structure.

Referring to Figures 1-4, preferably, the slider 2 further comprises a stable moving mechanism; and the stable moving mechanism cooperates with the supporting rail plates 11 to prevent the slider 2 from shaking while moving along the curvilinear guide rail 1.

Preferably, the stable moving mechanism is composed of two retaining wheels 23; the retaining wheels 23 are arranged at the rotating blocks 22; rims of the retaining wheels 23 are provided with annular wheel grooves 231; and two side edges 111 of the supporting rail plates 11 are respectively embedded in the wheel grooves 231 of one retaining wheel 23, to ensure the stability of the slider 2 when moving.

Here, the load of the bearing wheels 24 is lightened by the retaining wheels 23; under the guidance provided by the retaining wheels 23, the bearing wheels 24 may only be used for bearing the load, so that the structure is more stable, and the slider 2 may move more stably without shaking. Namely, each group of the wheel grooves 231 of the retaining wheels 23 at the front side and the rear side cooperate with the side edges 111 of the supporting rail plates 11 in an embedding mode, so that the retaining wheels 23 may only move smoothly along a curve direction of the supporting rail plates 11, thereby realizing the sliding of the slider 2 on the curvilinear guide rail 1; and meanwhile, the retaining wheels 23 provide certain support and bearing for the medical robot below.

Two groups of retaining wheels 23 in a left-and-right direction, i.e., two retaining wheels 23 at the front and back of the supporting rail plates 11, are adopted to make the retaining wheels 23 walk more stably.

The rotating blocks 22 are rotationally connected with the fixed base plate 21, to ensure that the rotating blocks 22 simultaneously rotates and changes the direction when walking to a radian region of the curvilinear guide rail 1, so that the rotating blocks can walk smoothly.

Here, the rotating blocks 22 may be U-shaped plates, wherein the bearing wheels 24 are arranged on inner walls of two sides of the U-shaped plates; and the retaining wheels 23 are arranged in two opening grooves on outer sides of the U-shaped plates.

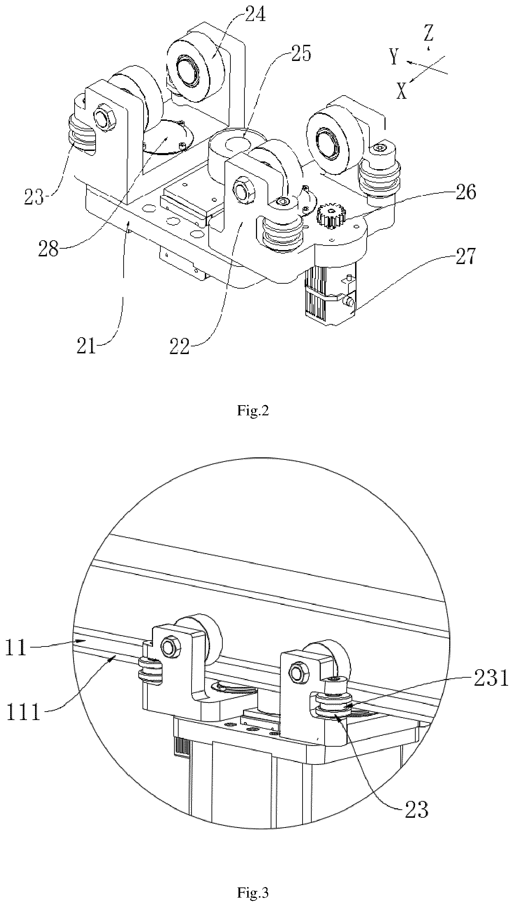

Referring to Figure 2, preferably, a brake 25 is provided at the top of the slider 2; and the brake 25 is adapted to stopping the slider 2 from moving along the curvilinear guide rail 1.

Here, the brake 25 is provided, so that when the brake 25 is activated, the slider 2 cannot continue to move on the curvilinear guide rail 1, thereby preventing the medical robot from unexpectedly moving and causing adverse consequences. The brake 25 here is referred to the prior art, and is subject to realizing the braking of the slider 2 on the curvilinear guide rail 1; for example, the brake may be a friction brake; and when the friction brake is activated, the braking is realized by a friction force between a braking part of the friction brake and the curvilinear guide rail 1, so as to keep a stop state.

Referring to Figures 2-4, preferably, the curvilinear guide rail 1 is provided with a rack 12; the rack 12 extends along the curvilinear guide rail 1; the slider 2 is provided with a gear 26 and a driving motor 27; the driving motor 27 is used for driving the gear 26 to rotate; and the gear 26 is meshed with the rack 12.

For example, the rack 12 may be arranged at the bottom of the supporting rail plates 11; and the gear 26 and the driving motor 27 are respectively arranged at the top and the bottom of one rotating block 22.

Here, a protruding surface may be extended from one side of one rotating block 22 far away from the other rotating block 22, thereby increasing the area of the rotating block 22, and providing a sufficient region position for allowing arrangement of the driving motor 27 and the gear 26, without hindering the rotation of the rotating block 22.

The gear 26 is driven by the driving motor 27 to rotate and is meshed with the fixed rack 12, so that the slider 2 "passively" moves along the curvilinear guide rail 1.

Referring to Figure 2, preferably, the retaining wheels 23 are rotationally connected with the rotating blocks 22; and the bearing wheels 24 are rotationally connected with the rotating blocks 22.

The bearing wheels 24 and the retaining wheels 26 are rotationally connected with the rotating blocks 22 respectively, so that when the bearing wheels 24 and the retaining wheels 23 walk on the supporting rail plates 11, sliding friction is converted into rolling friction, thereby reducing resistance.

Referring to Figure 1, the present embodiment further provides a medical robot, comprising the guide rail device, wherein the medical robot is connected to the bottom of the slider 2 in the guide rail device; for example, the medical robot is a laparoscopic surgical robot; the laparoscopic surgical robot comprises a robot body 3; and the robot body is connected to the bottom of the slider 2. The robot body 3 comprises a lifting rod 31, rotating arms 33, telescopic rods 34 and surgical manipulators 35, which are sequentially arranged from top to bottom, wherein the top of the lifting rod 31 is connected with the bottom of the slider 2; the lifting rod 31 and the telescopic rods 34 are vertically arranged; and the rotating arms 33 are horizontally arranged.

Through a lifting function of the lifting rod 31, the rotating arms 33, the telescopic rods 34 and the surgical manipulators 35 are suspended highly when the surgery is not needed; and the rotating arms 33, the telescopic rods 34 and the surgical manipulators 35 are moved down to a position slightly above the patient when the surgery is needed.

Referring to Figure 1, preferably, a plurality of rotating arms 33 are provided; an arm rotating bracket 32 is arranged at the bottom of the lifting rod 31; and on a plane perpendicular to the lifting rod 31, one ends of the plurality of rotating arms 33 are rotationally connected into an accommodating groove of the arm rotating bracket 32.

The plurality of rotating arms 33 are provided, so that a plurality of surgical manipulators 35 may be provided; and the surgery may be completed more flexibly and simply under the action of the plurality of surgical manipulators 35 in different azimuths.

Referring to Figure 1, preferably, the telescopic rods 34 are adapted to moving along the length direction of the rotating arms 33.

The surgical manipulators 35 are more flexible through lifting of the telescopic rods 34 within a small range, rotation of the rotating arms 33 and movement of the telescopic rods 34 on the rotating arms 33.

It should be noted that a specific structure of the robot body 3 may be referred to the prior art and will not be repeated here.

Here, compared with the floor-standing structure, such a ceiling-mounted structure occupies a small space, has all wires at high altitude, and has no cumbersome cables on the ground; through the lifting rod 31 on the Z-axis, the equipment may be lifted to the top when the surgery is not needed, thereby having little influence on walking and view of personnel; the spatial movement of the surgical manipulators 35 is realized by the sliding of the slider 2 on curvilinear guide rail 1 and mutual movement of various joints, so as to accurately and conveniently position a wound position of a wounded person on the hospital bed; compared with a cantilever robot, the robot provided by the invention has a more stable and simpler structure, stable operating, accurate positioning and better dexterity, and can safely complete more delicate and complex operations.

Although the present disclosure is described above, a protection scope of the present disclosure is not limited to the above. Those skilled in the art may make various changes and modifications without departing from spirit and scope of the present disclosure; and all the changes and modifications will fall within the protection scope of the invention.

## Claims

1. A guide rail device, comprising:
a curvilinear guide rail (1); and
a slider (2) slidably connected to the curvilinear guide rail (1), wherein the bottom of the slider (2) is adapted to connecting with a medical robot;
the curvilinear guide rail (1) comprises a first arc section guide rail (102) and a second arc section guide rail (104); and an arc opening direction of the first arc section guide rail (102) is opposite to that of the second arc section guide rail (104).

2. The guide rail device of claim 1, wherein the curvilinear guide rail (1) further comprises a first linear guide rail (101), a second linear guide rail (103) and a third linear guide rail (105) which are parallel to one another; the first linear guide rail (101), the first arc section guide rail (102), the second linear guide rail (103), the second arc section guide rail (104) and the third linear guide rail (105) are sequentially connected; an opening direction of the first arc section guide rail (102) faces one end of the second linear guide rail (103); and an opening direction of the second arc section guide rail (104) faces the other end of the second linear guide rail (103).

3. The guide rail device of claim 1, wherein the curvilinear guide rail (1) comprises a guide rail body (13) and a supporting rail plate (11); the supporting rail plate (11) is connected with the guide rail body (13); the supporting rail plate (11) has an elongated plate-like structure and extends along a length direction of the curvilinear guide rail (1); two supporting rail plates (11) are provided and respectively arranged at two sides of the guide rail body (13);
the slider (2) comprises a sliding mechanism, which is composed of two bearing wheels (24); the two bearing wheels (24) are respectively arranged at the tops of the two supporting rail plates (11); and the guide rail body (13) is arranged between the two bearing wheels (24), so as to prevent the slider (2) from being separated from the curvilinear guide rail (1).

4. The guide rail device of claim 3, wherein the slider (2) further comprises:
a fixed base plate (21); and
two rotating blocks (22), which are rotationally connected to the top of the fixed base plate (21) respectively; the bearing wheels (24) are arranged at the rotating blocks (22); and the supporting rail plates (11) are arranged between the bearing wheels (24) and the rotating blocks (22).

5. The guide rail device of claim 4, wherein the bearing wheels (24) are rotationally connected with the rotating blocks (22).

6. The guide rail device of claim 4, wherein the slider (2) further comprises a stable moving mechanism; the stable moving mechanism is composed of two retaining wheels (23); the retaining wheels (23) are arranged at the rotating blocks (22); rims of the retaining wheels (23) are provided with annular wheel grooves (231); and two side edges (111) of the supporting rail plates (11) are respectively arranged in the wheel grooves (231) of the two retaining wheels (23), to prevent the slider (2) from shaking while moving.

7. The guide rail device of claim 6, wherein the retaining wheels (23) are rotationally connected with the rotating blocks (22).

8. The guide rail device of claim 3, wherein the curvilinear guide rail (1) further comprises a rack (12); the rack (12) extends along the length direction of the supporting rail plates (11); the slider (2) further comprises a gear (26) and a driving motor (27); the driving motor (27) is used for driving the gear (26) to rotate; and the gear (26) is meshed with the rack (12).

9. The guide rail device of any one of claims 1-8, wherein a brake (25) is arranged at the top of the slider (2); and the brake (25) is adopted to stopping the slider (2) from moving along the curvilinear guide rail (1).

10. A medical robot, comprising the guide rail device of any one of claims 1-9.

11. The medical robot of claim 10, comprising a robot body (3), wherein the robot body (3) is connected with the slider (2) of the guide rail device.

12. The medical robot of claim 11, wherein the robot body (3) comprises a lifting rod (31), rotating arms (33), telescopic rods (34) and surgical manipulators (35) which are sequentially arranged from top to bottom; and the top of the lifting rod (31) is connected with the bottom of the slider (2).

13. The medical robot of claim 11, wherein the curvilinear guide rail (1) and the slider (2) of the guide rail device and the robot body (3) are sequentially arranged from top to bottom.
